# EUROPÄISCHE PATENTANMELDUNG

(11) **EP 1 103 256 A1**
(43) Veröffentlichungstag der Anmeldung: **30.05.2001**
(21) Anmeldenummer: 99123610.0
(22) Anmeldetag: 26.11.1999
(51) Int. Cl.: A61K 31/135, A61P 25/04

(54) **Verwendung von Ketamin zur Behandlung von neuroendokriner Immundysfunktion und algogenem Psychosyndrom**

(71) Anmelder: Böck, Claudius, Dr. med., 75181 Eutingen (DE); Zück, Gerhard, 75438 Knittlingen (DE)
(72) Erfinder: Böck, Claudius, Dr. med., 75181 Eutingen (DE); Zück, Gerhard, 75438 Knittlingen (DE)
(74) Vertreter: Patentanwälte Ruff, Beier und Partner

(57) **Zusammenfassung**

Die Erfindung betrifft eine Verwendung von Ketamin und ketaminhaltige Medikamente bzw. pharmazeutische Zusammensetzungen zur Behandlung der neuroendokrinen Immundysfunktion und des algogenen Psychosyndroms. Diese Krankheitsbilder zeichnen sich durch relativ breit gefächerte Symptome aus. Hierzu zählen vor allem Erschöpfungs- und Schmerzzustände. Unter anderem sind häufig psychische Symptome und Depressionen zu beobachten. Die erfindungsgemäße Verwendung von Ketamin stellt eine medikamentöse Therapie dieser Krankheitsbilder bereit, die eine deutliche Verbesserung der verschiedenen Symptome bis hin zu einem völligen Verschwinden der Symptome bewirkt. Bevorzugterweise wird Ketamin oral verabreicht. In einer weiteren Ausführungsform ist Ketamin zur topischen Verabreichung, vorzugsweise in Form einer Salbe oder eines Gels, vorgesehen.

## Beschreibung

Die Erfindung betrifft eine Verwendung von Ketamin und Medikamente zur Behandlung der neuroendokrinen Immundysfunktion und des algogenen Psychosyndroms.

Unter den Begriff neuroendokriner Immundysfunktion wird eine Anzahl von überwiegend vegetativ vermittelten Krankheitsbildern gefasst, die relativ breit gefächerte Symptomenkomplexe aufweisen. Hierzu zählen beispielsweise Spannungkopfschmerzen, Migräne, Colon irritabile, temporomandibuläres Schmerzsyndrom, das chronische Müdigkeitssyndrom oder das Fibromyalgiesyndrom. Erst in den letzten Jahren sind amerikanische Autoren dazu übergegangen, diese verschiedenen Krankheitsbilder bzw. Symptomenkomplexe in einem größeren pathogenetischen Rahmen zu sehen. Hierbei werden vor allein Fehlfunktionen im neuroendokrinologischen wie immunologischen Bereich als gemeinsame Ursache derartiger Krankheitsbilder verantwortlich gemacht (Peter A. Berg, Chronisches Müdigkeits- und Fibromyalgiesyndrom, Springer 1999).

Insbesondere das chronische Müdigkeitssyndrom und das Fibromyalgiesyndrom gehen häufig mit psychischen Symptomen und Depressionen einher. Die Kardinalsymptome sind Müdigkeit und/oder Erschöpfung bzw. ein generalisierter Muskelschmerz. Gleichzeitig treten auch eine Vielzahl funktioneller vegetativer, aber auch psychischer Störungen auf.

Ähnliche Symptome sind bei dem algogenen Psychosyndrom zu beobachten. Mit diesen Begriff wird eine komplexe und chronische Schmerzerkrankung beschrieben, die beim Patienten über viele Jahre hinweg entsteht (nach Prof. R. Wörz, Schmerzzentrum Bad Schönborn). Neben mehr oder minder für die Entstehung der Schmerzsymptomatik verantwortlichen organo-morphologischen Befunden, ist das algogene Psychosyndrom vor allem durch die sekundär auftretenden psychischen und psychosomatischen Veränderungen beim Patienten charakterisiert.

Die genannten Krankheitsbilder, vor allem die Fibromyalgie, sind in der ärztlichen Praxis ein häufiges, allerdings nicht immer erkanntes Krankheitsbild. Trotz der Übersetzung mit "Weichteil-Rheumatismus" ist das Fibromyalgie-Syndrom keine entzündliche oder gar rheumatische Erkrankung und ist deshalb im Blutbild und in Röntgenaufnahmen nicht nachweisbar. Die Erkrankung, die fast ausschließlich Frauen betrifft, äußert sich klinisch durch hinsichtlich Lokalisation und Schweregrad ständig wechselnden und vom Betroffenen oft mit pittoresken Schilderungen belegten Schmerzen, die von zahlreichen vegetativen Symptomen begleitet werden. Hierzu zählen beispielsweise Schlafstörungen, Müdigkeit, Abgeschlagenheit, Pelzigkeits- und Kribbelphänomene der Extremitäten, unspezifische Verdauungsprobleme, Gelenkschwellungen und Wassereinlagerungen, Ohrenprobleme, Schwitzneigung, trockene Schleimhäute in Augen und Mund, Druck- und Engegefühl über dem Brustkorb, Beklemmungs- und Angstattacken sowie depressive Verstimmungen. Dem Fibromyalgie-Syndrom sowie den anderen Krankheitsbildern, die unter den Sammelbegriff neuroendokriner Immundysfunktion gestellt werden, ist eine vegetative Dysregulation gemeinsam, die eine erhöhte Schmerzempfindlichkeit verursacht.

Die Ursachen des Fibromyalgie-Syndroms sind bis heute nicht eindeutig geklärt. Neben verschiedensten Auslösern wie z.B. Operationen, Unfälle, Infektionen und Umweltgiften spielen vor allem auch psychische Traumatisierungen und psychische Belastungen eine Rolle. Bei den meisten Erkrankten ist außerdem eine Überangepaßte Persönlichkeitsstruktur festzustellen, wobei die Betroffenen über Jahre hinweg mit hoher Leistungsmotivation und dem gleichzeitigen Verkennen der individuellen Belastbarkeitsgrenzen ihre körperlichen und seelischen Reserven aufgebraucht haben.

Die individuellen Symptome können bei den einzelnen Betroffenen relativ diffus sein. Dies dürfte auch der Grund sein, warum gängige Schmerzmedikamente bei dieser Erkrankung in praktisch allen Fällen völlig unwirksam sind und darüber hinaus häufig bei Fibromyalgie-Kranken erhebliche Nebenwirkungen hervorrufen. Dies trifft beispielsweise auf die sogenannten "NASR"-Medikamente wie beispielsweise Diclofenac zu, sowie auch auf Corticoide, Opiate bzw. Opioide und andere Schmerzmittel wie Novaminsulfon, Paracetamol oder Flupirtin.

Am Ort der wahrgenommenen Muskelschmerzen ist in aller Regel keine eigentliche organische Störung vorhanden. Die Krankheitsursache liegt also nicht im Gelenk oder in der Bandscheibe beispielsweise, sondern im vegetativen Nervensystem. Dies macht eine Therapie des Fibromyalgie-Syndroms sehr schwierig. Die Therapie wird noch zusätzlich dadurch erschwert, daß die Betroffenen und auch die Ärzte oft auf eine rein mechanisch/organisch orientierte Medizin geprägt sind. Weiterhin wird in nicht wenigen Fällen dem Patienten eine eingebildete oder simulierte Erkrankung unterstellt.

Eine sinnvolle Therapie des Fibromyalgie-Syndroms muß daher an mehreren Stellen ansetzen. Ein für den Verlauf der Krankeit entscheidender Punkt ist die Arbeit des Patienten mit sich selbst und mit seinem Lebensplan, d.h. das Vermeiden äußerer und innerer Krankheitsverstärker sowie das Erlernen eines gesunden Egoismus im Umgang mit der Umwelt und sich selbst. Der andere Schwerpunkt ist die ärztliche bzw. medikamentöse Behandlung, die zur Besserung der verschiedenen Symptome eingesetzt werden muß. Hierbei zeigt sich jedoch die besondere Problematik der unter dem Begriff neuroendokrine Immundysfunktion oder auch unter algogenem Psychosyndrom zusammengefassten Krankheitsbilder. Wie bei kaum einer anderen Krankheit entziehen sich bei diesen Krankheitsbildern die Symptome einer wirkungsvollen Medikamentation. Klassische Schmerz-Medikamente von Kortison bis hin zu Morphin sowie auch naturheilkundliche Verfahren bewirken kaum oder gar keine Linderung der auftretenden Symptome. Das sehr komplexe Zusammenspiel neuroendokriner und immunologischer Störungen, ausgelöst durch eine höchst individuelle Konstellation psychosozialer und psychovegetativer Belastungsfaktoren, erfordert eine intensive Beschäftigung mit einer geeigneten medikamentösen Therapie. Bisher konnten in dieser Hinsicht noch keine erfolgversprechenden Ergebnisse erzielt werden.

Die Erfindung stellt sich daher die Aufgabe, für die Behandlung der verschiedenen Krankheitsbilder, die unter den Begriff neuroendokrine Immundysfunktion sowie algogenes Psychosyndrom zu fassen sind, insbesondere für das Fibromyalgie-Syndrom, eine medikamentöse Therapie bereitzustellen, die eine deutliche Verbesserung der verschiedenen Symptome bis hin zu einem völligen Verschwinden der Symptome bewirkt. Diese Aufgabe wird gelöst durch die Verwendung des Stoffes Ketamin gemäß den Ansprüchen 1 und 2. Bevorzugte Ausführungsformen der erfindungsgemäßen Verwendung sind in den Ansprüchen 3 bis 16 dargestellt. Die Ansprüche 17 bis 20 betreffen entsprechende pharmazeutische Zusammensetzungen. Der Wortlaut sämtlicher Ansprüche wird hiermit durch Bezugnahme zum Inhalt der Beschreibung gemacht.

Gegenstand der Erfindung ist die Verwendung von Ketamin zur Herstellung einer pharmazeutischen Zusammensetzung oder eines Medikaments zur Behandlung von neuroendokriner Immundysfunktion oder algogenem Psychosyndrom. Weiterer Gegenstand der Erfindung ist die Verwendung von Ketamin zur Behandlung dieser genannten Krankheiten.

Unter Ketamin im Sinne der Erfindung ist die Hydrochloridform zu verstehen (2 (2-Chlorphenyl)-2-methylamino-cyclohexanonhydrochlorid). Hierbei handelt es sich um eine weiße, kristalline Substanz mit bitterem Geschmack und schwachem, typischen Geruch. Der Einsatz von Ketamin als medikamentöser Wirkstoff am Menschen fand erstmals in den USA im Jahr 1964 statt. In Deutschland wird die Substanz seit nahezu 30 Jahren verwendet. In erster Linie gilt Ketamin als Anästhetikum. Daneben hat Ketamin auch eine hypnotische Wirkung, die in Ausnahmesituationen den Einsatz als Monoanästhetikum erlaubt. In der Humanmedizin konnte sich der standardmäßige Einsatz von Ketamin als Anästhetikum jedoch nie durchsetzen, da die Verwendung dieses Medikaments mit einer Vielzahl von Nebenwirkungen einhergeht. Hierzu zählen Kreislaufeffekte sowie alptraumhafte Erlebnisvorstellungen in der Aufwachphase, so daß Ketamin nur in Kombination mit sedierenden Komponenten eingesetzt werden kann. Lediglich in der Notfallmedizin kommt Ketamin bei bestimmten Indikationen zum Einsatz. Ansonsten haben sich andere, überwiegend gasförmige (= volatile), in jüngster Zeit aber auch wieder verstärkt intravenöse Anästhetika durchgesetzt. Intravenöses Ketamin zur Narkoseeinleitung oder auch in der Notfallmedizin hat eine Außenseiterstellung und spielt heute zahlenmäßig kaum eine Rolle. Im Gegensatz zur Humanmedizin wird Ketamin in der Tiermedizin häufiger eingesetzt.

Der anästhetische Effekt von Ketamin ist bisher weder elektrophysiologisch noch biochemisch vollständig geklärt.

Neben seiner Wirkung als Anästhetikum entfaltet Ketamin auch eine analgetische, also schmerzlindernde Wirkung. Allerdings wurde Ketamin bislang nicht in nennenswertem Umfang zur Therapie chronischer Schmerzen eingesetzt. Es gibt jedoch einzelne Berichte, daß Ketamin beispielsweise erfolgreich zur Therapie von Stumpfschmerzen, Tumorschmerzen oder von postherpetischer Neuralgie verwendet wurde.

Die von der Firma PARKE-DAVIS herausgegebene Produktmonographie des S-Enantiomers von Ketamin (Ketanest®-S) weist darauf hin, daß starke chronische Schmerzen durch Ketamin zu beeinflussen sind. In diesem Zusammenhang erfolgt auch ein Hinweis auf das Krankheitsbild der Fibromyalgie.

Ein zusammenfassender Artikel von B. Wiedemann (Der Schmerz 1997, 11:276-281) stellt verschiedene Publikationen zusammen, die Ketamin zur Therapie chronischer Schmerzen einsetzen. Die Diagnosen, bei denen Ketamin zum Einsatz kam, waren postherpetische Neuralgie chronischer posttraumatischer Schmerz mit Allodynie oder zentraler Dysästhesie (Sensibilitätsstörung), chronischer Rückenschmerz bei Osteoporose und Tumorschmerz.

Bei allen bisherigen Anwendungen von Ketamin steht die anästhetische Wirkung bzw. der rein analgetische Effekt im Vordergrund. Die Erfindung basiert nun auf dem überraschenden Ergebnis, daß bei den Krankheitsbildern neuroendokrine Immundysfunktion und algogenes Psychosyndrom, bei denen Schmerzempfindungen nur eines von vielen Symptomen ist, durch die Verabreichung von Ketamin eine Besserung von vielen oder sogar allen der verschiedenen Beschwerden zu erreichen ist. Neben einer Verbesserung des Schinerzniveaus werden beispielsweise depressive Verstimmungen oder Angstzustände deutlich gelindert. Auch andere vegetative Symptome wie Abgeschlagenheitsgefühl, Gelenkschwellungen oder Schlafstörungen werden durch die Verabreichung von Ketamin verbessert. Die erfindungsgemäße Verwendung von Ketamin hat daher den entscheidenden Vorteil, die relativ diffusen Symptome von Krankheiten wie dem Fibromyalgie-Syndrom oder anderen unter neuroendokriner Immundysfunktion zu fassende Krankheiten mit einem einzigen Wirkstoff behandeln zu können. Außerdem ist die analgetische Wirksamkeit von Ketamin bei dieser Art von Erkrankung anderen Schmerzmitteln außerordentlich deutlich überlegen.

In einer bevorzugten Ausführungsform der erfindungsgemäßen Verwendung wird razemisches Ketaminhydrochlorid eingesetzt. Ein derartiges Gemisch der beiden Enantiomere (S)- und (R)-Ketamin ist frei auf dem Markt erhältlich (Rezeptursubstanz). Die pharmakologische Wirkung von (S)-Ketamin entspricht weitgehend dem des razemischen Gemisches. Die einzelnen Effekte sind bei unterschiedlicher quantitativer Ausprägung qualitativ insgesamt vergleichbar. Im allgemeinen sind die Wirkungen des (S)-Ketamins jedoch in etwa dreifach höher als die der (R)-Form bzw. doppelt so hoch wie die des Razemats. Daher wird in einer besonders bevorzugten Ausführungsform Ketamin in Form des S-Enantiomers eingesetzt.

Die Verwendung des S-Enantionmers von Ketamin hat weiterhin den entscheidenden Vorteil, daß zentrale Nebenwirkungen wie beispielsweise Benommenheit oder Beklemmungen, im Vergleich mit dem Razemat, deutlich schwächer und bei richtig eingesteller Dosierung völlig vernachlässigbar auftreten.

In einer bevorzugten Ausführungsform der Erfindung ist es vorgesehen, Ketamin in fester Form zu verabreichen. Abgesehen von Rezeptursubstanzen ist Ketamin bisher lediglich in gelöster Form als Injektionslösung erhältlich. Erfindungsgemäß ist die feste Form, beispielsweise als Kapseln oder Tabletten, vorgesehen, da hierdurch die Verabreichung wesentlich erleichtert wird. Die Patienten sind selbständig in der Lage, das Medikament einzunehmen und sind nicht auf ärztliche Hilfe bei den Injektionen angewiesen. Die Dosierung des Wirkstoffs ist z.B. mit Tabletten völlig unproblematisch, da die Anzahl und die Wirkstoffkonzentration der Kapseln oder Tabletten variiert werden kann.

In einer bevorzugten Ausführungsform ist Ketamin zur oralen Verabreichung vorgesehen. Der Wirkstoff kann hier in Tabletten oder Kapseln eingearbeitet sein. Die Verabreichung ist bei dieser Form völlig unproblematisch. In einer weiteren Ausführungsform der Erfindung ist Ketamin zur rektalen Verabreichung, beispielsweise in Form von Zäpfchen, vorgesehen. Ein Vorteil hierbei ist, daß der Wirkstoff auf diese Weise schneller in den Organismus, insbesondere in die Blutbahn, aufgenommen wird, und so deutlich schneller wirken kann.

Ketaminhydrochlorid läßt sich mit Kapselfüllstoffen wie Manitol oder Laktose problemlos verarbeiten. Bei Supositorienzubereitungen sind hydrophile Grundlagen (beispielsweise Polyethylenglykol) bevorzugt. Hierdurch wird eine schnelle Wirkstoffabgabe gewährleistet. Von daher kann Ketaminhydrochlorid nach gängigen pharmazeutischen Methoden für die Verabreichung vorbereitet werden.

Für die Verabreichung in fester Form ist eine Tagesdosis von etwa 20 bis etwa 200 mg vorgesehen. Besonders bevorzugt ist eine Tagesdosis von etwa 40 bis etwa 150 mg. Diese Angaben beziehen sich auf einen Patienten durchschnittlicher Größe und Gewicht, wobei es sich insbesondere um weibliche Patienten handelt. Die jeweilige Tagesdosis ist auf den entsprechenden Patienten einzustellen und hängt von der Schwere der Symptome und der Reaktion des Patienten auf den Wirkstoff ab. Bevorzugterweise wird diese optimale Tagesdosis zu Beginn einer Therapie vom behandelnden Arzt ermittelt.

Vorteilhafterweise wird die Tagesdosis in mehreren Einzeldosen über den Tag verteilt verabreicht. Beispielsweise kann der Wirkstoff bis zu sechmal am Tag verabreicht werden, um eine möglichst optimale gleichmäßige Wirkstoffkonzentration beim Patienten zu erreichen. Besonders bevorzugt ist eine drei- und/oder viermalige Verabreichung pro Tag. Dies ist für den Patienten besonders angenehm zu handhaben, da beispielsweise zu jeder Mahlzeit eine Tablette eingenommen werden kann. Die mehrfache Verabreichung von Ketamin über den Tag zeigt im allgemeinen eine bessere Wirkung als die Verabreichung der Tagesdosis in größeren Einzeldosen. Schon durch eine viermalige Ketamin-Gabe pro Tag wird im allgemeinen ein besserer Effekt als bei einer dreimaligen Gabe pro Tag erzielt.

Vorzugsweise enthält jede Einzeldosis etwa 5 bis etwa 100 mg des Wirkstoffs, insbesondere etwa 10 oder etwa 50 mg pro Einzeldosis. Es ist möglich, daß jede Einzeldosis gleiche Wirkstoffkonzentration enthält. Andererseits kann es auch vorteilhaft sein, beispielsweise gegen Abend eine höhere Einzeldosis, beispielsweise etwa 50 mg, einzunehmen, um über die Nacht hinweg eine entsprechend hohe Wirkstoffkonzentration im Organismus zu gewährleisten.

In einer besonders bevorzugten Ausführungsform der Erfindung ist Ketamin zur topischen Verabreichung, also lokal, vorgesehen. Dies kann bevorzugt sein, wenn sich die auftretenden Schmerzen lokalisieren lassen, indem sie beispielsweise an bestimmten Gelenken auftreten. Vorteilhafterweise wird der Wirkstoff in Form einer Salbe oder eines Gels bereitgestellt, die oder das dann auf die entsprechenden Hautstellen aufgetragen werden kann.

Das Löslichkeitsverhalten des Wirkstoffs ist relativ unproblematisch. In Wasser ist Ketaminhydrochlorid mit etwa 200 mg/ml gut löslich, ebenso in Methanol. Die Löslichkeit in wasserhaltigen Creme- und Gelgrundlagen ist ebenso gut wie in alkoholhaltigen Grundlagen. Daher ist die Verarbeitung in Salben- oder Gelform nach üblichen Methoden möglich. Besonders bevorzugt ist eine Liposomen-Zubereitung, die schnell in die Haut und das darunterliegende Bindegewebe einziehen kann, so daß der Wirkstoff seine Wirkung sehr schnell entfaltet. Daher kann es bereits nach wenigen Minuten der topischen Applikation zu einer merklichen Schmerzreduktion am Ort des Salben- oder Gelauftrages kommen.

Bevorzugterweise ist bei der topischen Verabreichung eine Tagesdosis von etwa 5 bis etwa 500 mg pro Patient vorgesehen. Vorteilhafterweise wird etwa 50 bis etwa 150 mg pro Patient aufgetragen. Ein entscheidender Vorteil bei der topischen Verabreichung ist, daß der Patient je nach Beschwerden die Verabreichung selbst vornehmen und dosieren kann. Auf diese Weise kann die Wirkstoffkonzentration je nach Bedürfnis optimal eingestellt werden.

Für die Verabreichung in Salbenform kann beispielsweise eine Wirkstoffkonzentration von etwa 100 mg/ml eingestellt werden. Bei einer solchen Konzentration sollte je nach Bedarf etwa zweimal 0,5 ml pro Tag auf die betroffenen Stellen aufgetragen werden.

In einer besonders bevorzugten Ausführungsform der Erfindung ist eine kombinierte Verabreichungsform vorgesehen. Hierbei wird der Wirkstoff zum einen in gelöster und zum anderen in fester Form verabreicht, wobei eine Kombination von intravenöser und oraler Verabreichung vorgesehen ist. Die medikamentöse Therapie kann mit der intravenösen Verabreichung der gelösten Form begonnen werden. Durch ein-, zwei- oder dreimalige Ketamin-Infusionen pro Woche wird dabei die nötige Frequenz der Therapie vom Arzt festgestellt und entsprechend eingestellt. Eine geeignete Wochendosis kann dabei auf etwa 1 bis etwa 100 mg, insbesondere auf etwa 10 bis etwa 50 mg Wirkstoff pro Patient eingestellt werden. Beispielsweise kann eine dreimalige Infusion pro Woche mit jeweils etwa 5 mg Ketamin pro Infusion bei alleiniger intravenöser Applikation eine sehr gute Wirksamkeit bei praktisch fehlenden Nebenwirkungen entfalten.

Hierbei bleibt jedoch immer zu berücksichtigen, daß bei der optimalen wirksamen Dosis eine hohe individuelle Schwankungsbreite zu beobachten ist. Es kann unter dem Gesichtspunkt, daß Ketamin bei relativer Überdosierung somnolente, hypnotische, schließlich auch narkoseeinleitende Effekte besitzt, sehr wichtig sein, daß zum Anfang der Therapie die entsprechend geeignete Dosis vom Arzt festgestellt wird.

Bei der intravenösen Verabreichung kann die Wochendosis vorzugsweise in bis zu fünfmaligen einzelnen Infusionen pro Woche verabreicht werden. Hierbei ist natürlich zu bedenken, daß der Patient für die Infusion in der Regel die Arztpraxis aufsuchen muß. Pro Infusion wird vorteilhafterweise etwa 1 bis etwa 10 mg Wirkstoff verabreicht.

Es ist bevorzugt, daß nach der intravenösen Verabreichungsphase die Applikation im weiteren oral erfolgt. In dieser Phase der Therapie ist es also nicht mehr notwendig, daß der Patient so häufig den Arzt aufsuchen muß. Die geeignete Dosis für die orale Verabreichung muß selbstverständlich auch vom Arzt eingestellt werden. Hierbei ist zu berücksichtigen, daß die Wirkstoffdosis bei einer oralen Verabreichung in der Regel höher liegt als bei der intravenösen Verabreichung. Im Verlauf einer längerfristigen Therapie kann es vorteilhaft sein, die Tagesdosis dem Krankheitsverlauf anzupassen, indem beispielsweise die Dosis Schritt für Schritt reduziert wird.

In einer besonders bevorzugten Ausführungsform der erfindungsgemäßen Verwendung ist Ketamin für eine retardierte Verabreichung vorgesehen. Hierbei wird der Wirkstoff in einer solchen Form verabreicht, daß er langsam bzw. verzögert im Organismus freigesetzt wird. Eine retardierte orale Wirkstofffreisetzung kann beispielsweise durch eine Kombination mit speziellen Zellulose-Derivaten erreicht werden. Auch die rektale Verabreichungsform ist für eine retardierte Wirkstofffreisetzung geeignet. Durch eine derartige verzögerte Wirkstofffreisetzung wird gewährleistet, daß eine relativ gleichmäßige Wirkstoffkonzentration im Patienten erreicht wird, so daß unter Umständen die mehrfache Aufnahme von beispielsweise Tabletten oder Kapseln pro Tag vermieden werden kann. So kann es beispielsweise ausreichend sein, durch eine tägliche Kapsel mit retardierter Wirkstofffreisetzung die erforderliche Wirkstoffkonzentration über den Zeitverlauf zu gewährleisten. Dies ist besonders bei einer längerfristigen Behandlung zu bevorzugen, da hier das tägliche mehrfache, oftmals als lästig empfundene Einnehmen von Kapseln, Tabletten o.ä. vermieden werden kann.

Durch die erfindungsgemäße Verwendung von Ketamin ist es erstmals möglich, die Krankheitsbilder der neuroendokrinen Immundysfunktion oder des algogenen Psychosyndroms, insbesondere das Fibromyalgie-Syndrom oder das chronische Müdigkeitssyndrom, medikamentös sinnvoll und wirksam zu behandeln. Die Erfindung ermöglicht eine längerfristige Therapie, die den betroffenen Patienten deutliche Linderung aller Beschwerden und damit eine signifikante Verbesserung der Lebensqualität bringt. Die Verwendung von Ketamin ist für eine Behandlung über Monate oder sogar Jahre geeignet, da bei der relativ geringen Wirkstoffkonzentration keine Nebenwirkungen zu beobachten sind.

Weiterhin umfasst die Erfindung pharmazeutische Zusammensetzungen oder Medikamente, die den Wirkstoff Ketaminhydrochlorid in einer wirksamen Menge umfassen. Bei diesen pharmazeutischen Zusammensetzungen handelt es sich um Salben oder Gele, Tabletten oder Kapseln oder um Zusammensetzungen, die für eine rektale Verabreichung geeignet sind, beispielsweise um Zäpfchen bzw. Suppositorien. Die pharmazeutischen Zusammensetzungen sind dadurch gekennzeichnet, daß sie Ketamin in einer wirksamen Menge sowie mindestens einen pharmazeutisch akzeptablen Träger umfassen. Die pharmazeutischen Zusammensetzungen werden nach dem Fachmann bekannten Methoden hergestellt. Beispielhafte Einzelheiten hierzu sind dem nachfolgenden Beispiel zu entnehmen. In bevorzugten Ausführungsformen der pharmazeutischen Zusammensetzungen ist eine retardierte Abgabe der Wirkstoffe im Organismus vorgesehen. Diese Retard-Formen der pharmazeutischen Zusammensetzungen werden ebenfalls nach üblichen Methoden hergestellt.

Die beschriebenen Merkmale sowie weitere Merkmale der Erfindung ergeben sich aus der folgenden Beschreibung von Beispielen in Verbindung mit den Unteransprüchen, wobei jedes der einzelnen Merkmale für sich oder in Kombination mit anderen verwirklicht sein kann.

### Beispiele

### 1. Behandlungsbeispiel

Fünf Patientinnen mit folgenden Symptomen: Müdigkeits- und Erschöpfungssymptome, Spannungskopfschmerzen, Migräne-Dispositon, Muskel- und Weichteilschmerzen über mindestens 5 Jahre, hinsichtlich Lokalisation und Schweregrad wechselnde Schmerzen, Kriterium des "wide-spread pain" erfüllt, nächtliche Schlafstörungen, Paraesthesien der Extremitäten, pseudo-pectanginöse Beschwerden, Irritation der Atemwege, unspezifische allergische Anamnese, sub-depressive bis depressive Episoden, unspezifische Verdauungsstörungen, Angst- und Panikattacken, wurden über einen Zeitraum von insgesamt 26 Wochen mit Ketamin behandelt. Zunächst erfolgte eine intravenöse Verabreichungsphase, wobei den Patientinnen eine Wochendosis von 25 mg Ketaminhydrochlorid (Razemat) durch drei Infusionen pro Woche verabreicht wurde. Nach einer Woche der intravenösen Verabreichung wurde die Therapie auf eine orale Applikation des Wirkstoffes umgestellt. Hierbei wurden Kapseln mit je 10 mg Ketaminhydrochlorid (Razemat) viermal pro Tag oral eingenommen. Schon in der ersten intravenösen Verabreichungsphase konnte eine deutliche Besserung der Symptome beobachtet werden. Depressive Verstimmungen, Abgeschlagenheit, Tagesmüdigkeit oder Schlafstörungen wurden signifikant gelindert. Allgemein konnte eine Stabilisierung der Psyche, eine Reduktion angst- und panikgeprägter Phasen sowie eine Antriebssteigerung untertags festgestellt werden. Das Schmerzniveau senkte sich drastisch, so daß sich die Patientinnen z.T. völlig schmerzfrei fühlten. Insgesamt trat eine deutliche Verbesserung des Allgemeinbefindens und des Lebensgefühls ein. Dieses Ansprechen auf die Behandlung mit Ketaminhydrochlorid setzte bereits nach wenigen Tagen der intravenösen Verabreichung des Wirketoffes ein und hielt bis zum Ende der durchgeführten Studie an. Nebenwirkungen konnten keine beobachtet werden.

### 2. Rezepturen für Kapseln

a) Ketaminhydrochlorid 10, 20, 30 oder 50 mg pro Dosis Füllstoff NRF = Mannitol 99,5 %, Aerosil 0,5 % quantum satis
b) Ketaminhydrochlorid 10, 20, 30 oder 50 mg pro Dosis Lactose 95 %, Aerosil 0,5 % quantum satis
   - Abfühlung in Gelatine-Kapseln Gr. 3, Füllmenge 0,18 g
c) Ketaminhydrochlorid 25 mg pro Dosis Methylhydroxypropylcellulose (Methocel E4M Premium ER USP XXIII) 100 mg Lactose quantum satis
   - Beispiel für eine retardierte Darreichungsform: Abfüllung in Gelatinekapseln Gr. 2, Füllmenge 0,26 g

### 3. Rezeptur für Suppositorien

- Ketaminhydrochlorid piv.subt. 20 mg pro Dosis PEG 400 / PEG 6000 3:2 quantum satis Suppositoriengiessform 2 g

### 4. Rezepturen für Gele

a) Ketaminhydrochlorid 10 %
   Lipodermin-Konzentrat HT (ungeladenes Liposomen-Gel, ethanolhaltig) 90 %
   Tubenabfüllung
b) Ketaminhydrochlorid 10 %
   Natipide II PG (unbeladenes Liposomen-Gel, ethanolfrei) 90 %
   Stabilisierung mit 0,1 % Xanthan u.U. notwendig
c) Ketaminhydrochlorid 10 %
   PLO-Grundlage 90 %
   PLO = Pluronic-Lecithin-Organogel = Lecithin e Sojae/Isopropylmyristat 1:1 20-40 % + Poloxamer 407 20 % in wäseriger Lösung 80-60 %
   - Konservierungen der Formulierungen b) und c) empfiehlt sich z.B. mit 0,2 % Sorbinsäure.

## Patentansprüche

1. Verwendung von Ketamin (Ketaminhydrochlorid) zur Herstellung einer pharmazeutischen Zusammensetzung oder eines Medikaments zur Behandlung von neuroendokriner Immundysfunktion oder des algogenen Psychosyndroms.

2. Verwendung von Ketamin zur Behandlung von neuroendokriner Immundysfunktion oder des algogenen Psychosyndroms.

3. Verwendung nach Anspruch 1 oder Anspruch 2, dadurch gekennzeichnet, daß das Ketamin als Racemat verwendet wird.

4. Verwendung nach Anspruch 1 oder Anspruch 2, dadurch gekennzeichnet, daß das S-Enantiomer von Ketamin verwendet wird.

5. Verwendung nach einem der vorhergehenden Ansprüche, dadurch gekennzeichnet, daß das Ketamin zur Verabreichung in fester Form vorgesehen ist.

6. Verwendung nach einem der vorhergehenden Ansprüche, insbesondere nach Anspruch 5, dadurch gekennzeichnet, daß das Ketamin zur oralen Verabreichung vorgesehen ist.

7. Verwendung nach einem der vorhergehenden Ansprüche, insbesondere nach Anspruch 5, dadurch gekennzeichnet, daß Ketamin zur rektalen Verabreichung vorgesehen ist.

8. Verwendung nach einem der vorhergehenden Ansprüche, dadurch gekennzeichnet, daß eine Tagesdosis von etwa 20 bis etwa 200 mg, insbesondere etwa 40 bis etwa 150 mg pro Patient vorgesehen ist.

9. Verwendung nach einem der vorhergehenden Ansprüche, dadurch gekennzeichnet, daß das Ketamin für eine einbis sechsmalige, inbesondere drei- und/oder viermalige, Verabreichung pro Tag vorgesehen ist, wobei vorzugsweise etwa 5 bis etwa 100 mg, insbesondere etwa 10 und/oder etwa 50 mg pro Einzeldosis vorgesehen ist.

10. Verwendung nach nach einem der Ansprüche 1 bis 5, dadurch gekennzeichnet, daß das Ketamin zur topischen Verabreichung, vorzugsweise in Form einer Salbe oder eines Gels, vorgesehen ist.

11. Verwendung nach Anspruch 10, dadurch gekennzeichnet, daß eine Tagesdosis von etwa 5 bis etwa 500 mg, insbesondere etwa 50 bis etwa 150 mg pro Patient vorgesehen ist.

12. Verwendung nach einem der Ansprüche 1 bis 4, dadurch gekennzeichnet, daß das Ketamin zur Verabreichung in gelöster und in fester Form vorgesehen ist, wobei eine Kombination von intravenöser und oraler Verabreichung vorgesehen ist.

13. Verwendung nach Anspruch 12, dadurch gekennzeichnet, daß für den Beginn einer Therapie eine intravenöse und für die weitere Therapie eine orale Verabreichung von Ketamin vorgesehen ist.

14. Verwendung nach Anspruch 13, dadurch gekennzeichnet, daß für die intravenöse Verabreichung eine Wochendosis von etwa 1 bis etwa 100 mg, insbesondere von etwa 10 bis etwa 50 mg pro Patient vorgesehen ist.

15. Verwendung nach Anspruch 14, dadurch gekennzeichnet, daß für die Verabreichung der Wochendosis eine ein- bis fünfmalige, insbesondere dreimalige, Infusion pro Woche vorgesehen ist, wobei vorzugsweise etwa 1 bis etwa 10 mg, insbesondere etwa 5 mg pro Infusion vorgesehen sind.

16. Verwendung nach einem der vorhergehenden Ansprüche, dadurch gekennzeichnet, daß Ketamin für eine retardierte Verabreichung vorgesehen ist.

17. Pharmazeutische Zusammensetzung oder Medikament in Form einer Salbe, dadurch gekennzeichnet, daß sie Ketamin in einer wirksamen Menge und mindestens einen pharmazeutisch akzeptablen Träger umfaßt.

18. Pharmazeutische Zusammensetzung oder Medikament in Form einer Tablette oder Kapsel, dadurch gekennzeichnet, daß sie Ketamin in einer wirksamen Menge und mindestens einen pharmazeutisch akzeptablen Träger umfaßt.

19. Pharmazeutische Zusammensetzung oder Medikament in Form eines Suppositoriums, dadurch gekennzeichnet, daß es Ketamin in einer wirksamen Menge und mindestens einen pharmazeutisch akzeptablen Träger umfaßt.

20. Pharmazeutische Zusammensetzung nach einem der Ansprüche 17 bis 19, dadurch gekennzeichnet, daß das Ketamin für eine retardierte Wirkstoffabgabe aufbereitet ist.
